# EUROPEAN PATENT SPECIFICATION

(11) **EP 0 382 095 B1**
(45) Date of publication and mention of the grant of the patent: **10.05.1995**
(21) Application number: 90102014.9
(22) Date of filing: 01.02.1990
(51) Int. Cl.: C11D 7/50, C23G 5/028, C07C 19/08

(54) **Azeotropic or azeotropic-like composition of hydrochlorofluoropropane**
Azeotrope oder azeotropähnliche Zusammensetzung auf der Basis von Wasserstoff enthaltenden Chlorfluorkohlenwasserstoffen
Mélange azéotropique ou semblable à un mélange azéotropique à base d'hydrocarbures hydrogénés, chlorés et fluorés

(30) Priority: 06.02.1989 JP 25641/89; 06.02.1989 JP 25652/89; 06.02.1989 JP 25682/89; 06.02.1989 JP 25683/89; 18.04.1989 JP 96464/89
(43) Date of publication of application: 16.08.1990
(73) Proprietor: ASAHI GLASS COMPANY LTD., Chiyoda-ku Tokyo 100 (JP)
(72) Inventor: Samejima, Shunichi, Nakano-ku, Tokyo (JP); Kitamura, Kenroh, Fujisawa-shi, Kanagawa-ken (JP); Watanabe, Naohiro, Chiba-shi, Chiba-ken (JP); Asano, Teruo, Yokohama-shi, Kanagawa-ken (JP); Kamimura, Toru, Chiba-ken (JP)
(74) Representative: Wächtershäuser, Günter, Prof. Dr.

(56) References cited:
- EP-A- 0 347 924
- EP-A- 0 381 216
- US-A- 3 080 430
- US-A- 3 476 819
- PATENT ABSTRACTS OF JAPAN, vol. 13, no. 255 (C-606)[3603], 13th June 1989; & JP-A-1 60 694
- PATENT ABSTRACTS OF JAPAN, vol. 13, no. 124 (C-580)[3472], 27th March 1989;& JP-A-63 295 699

## Description

The present invention relates to a novel hydrochlorofluoropropane azeotropic or azeotropic-like composition which can be used as a chlorofluorocarbon alternative and which has excellent properties as a solvent and so on.

Chlorofluorocarbon compounds (hereinafter referred simply as CFCs) have little toxicity and are, in many cases, non-flammable and chemically stable. Various CFCs having different boiling points are available. By virtue of such properties, 1,1,2-trichloro-1,2,2-trifluoroethane (R113) is used as a solvent or a blowing agent; trichloromonofluoromethane (R11) is used as a blowing agent or a propellant; and dichlorodifluoromethane (R12) is used as a propellant or a refrigerant.

Chemically stable R11, R12 and R113 have long lifetime in the troposphere and reach the stratosphere, where they will be dissociated by solar radiation to release chlorine radicals, which initiate a chain reaction with ozone and deplete the ozone layer. Accordingly, the regulations for limiting the use of such conventional CFCs have been implemented. Therefore, a research has been actively conducted to develop a CFC alternative which scarcely depletes the ozone layer.

In a prior, non pre-published patent application by the present applicant (EP-A-0 347 924) halogenated hydrocarbon solvents are disclosed consisting essentially of hydrogen-containing chlorofluoropropanes, or mixtures thereof.

In another non pre-published prior patent application of the present applicant (EP-0 381 216) hydrochlorofluorocarbon azeotropic or azeotropic-like mixtures are described which contain one or more hydrochlorofluoropropanes and at least one member selected from halogenated hydrocarbons other than hydrochlorofluoropropane, hydrocarbons and alcohols.

It is an object of the present invention to provide a mixture comprising hydrochlorofluoropropanes having 3 carbon atoms, which has various excellent properties equal to conventional CFCs and which is useful as a CFC alternative.

The present invention provides in one embodiment an azeotropic or azeotropic-like composition which comprises 3,3-dichlorol-1,1,1,2,2-pentafluoropropane (R225ca), 1,3-dichloro-1,1,2,2,3-pentafluoropropane (R225cb) and 3-chloro-1,1,2,2-tetrafluoropropane (R244ca).

Other compositions of the present invention are defined in independent Claims 4, 5, 8, 11, 13, and 15.

The composition of the present invention is non-flammable and may take a form of an azeotropic composition or an azeotropic-like composition. Particularly when used as a solvent, it provides properties equal or superior to conventional 1,1,2-trichlorotrifluoroethane (R113). Therefore, it is very useful as an alternative for R113. Further, no substantial change was observed in the composition when boiling or evaporating. Therefore, it may be used in the same manner as a conventional single CFC, whereby it has a merit in that no substantial change in the conventional technique is required.

The hydrochlorofluoropropanes used in the present invention include the following compounds:
CF₃CF₂CHCℓ₂(R225ca)
CCℓF₂CF₂CHCℓF(R225cb)
CH₃CF₂CCℓ₂F(R243cc)
CHF₂CF₂CH₂Cℓ(R244ca)
CH₂FCF₂CHCℓF(R244cb)
The composition comprising R225ca, R225cb and R244ca of the present invention usually comprises from 12 to 92% by weight of R225ca, from 3 to 77% by weight of R225cb and from 3 to 69% by weight of R244ca, preferably from 51 to 64% by weight of R225ca, from 4 to 16% by weight of R225cb and from 26 to 39% by weight of R244ca. More preferably, it is an azeotropic composition comprising 58% by weight of R225ca, 10% by weight of R225cb and 32% by weight of R244ca.

The composition comprising R225ca and R225cb of the present invention has excellent azeotropic-like characteristics over the entire range of the blend ratio of R225ca and R225cb. The composition of the present invention comprises R225ca and R225cb in weight ratios of 80/20 to 19/81.

The composition comprising R225cb and R244ca of the present invention usually comprises from 27 to 67% by weight of R225cb and from 33 to 73% by weight of R244ca, preferably from 37 to 57% by weight of R225cb and from 43 to 63% by weight of R244ca. More preferably, it is an azeotropic composition comprising 47% by weight of R225cb and 53% by weight of R244ca.

The composition comprising R225ca and R244ca of the present invention usually comprises from 52 to 92% by weight of R225ca and from 8 to 48% by weight of R244ca, preferably from 62 to 82% by weight of R225ca and from 18 to 38% by weight of R244ca. More preferably, it is an azeotropic composition comprising 72% by weight of R225ca and 28% by weight of R244ca.

When R225ca and R243cc are used, the composition of the present invention usually comprises from 56 to 96% by weight of R225ca and from 4 to 44% by weight of R243cc, preferably from 66 to 86% by weight of R225ca and from 14 to 34% by weight of R243cc. More preferably, it is an azeotropic composition comprising 76% by weight of R225ca and 24% by weight of R243cc.

When R225cb and R244cb are employed, the composition usually comprises from 47 to 87% by weight of R225cb and from 13 to 53% by weight of R244cb, preferably from 57 to 77% by weight of R225cb and from 23 to 43% by weight of R244cb. More preferably, it is an azeotropic composition comprising 67% by weight of R225cb and 33% by weight of R244cb.

When R225cb and R243cc are used, the composition usually comprises from 43 to 83% by weight of R225cb and from 17 to 57% by weight of R243cc, preferably from 53 to 73% by weight of R225cb and from 27 to 47% by weight of R243cc. More preferably, it is an azeotropic composition comprising 63% by weight of R225cb and 37% by weight of R243cc.

To the composition of the present invention, other components may further be incorporated depending upon its application. For example, when the composition is used as a solvent, it may suitably contain a hydrocarbon such as pentane, isopentane, hexane, isohexane, neohexane, heptane, isoheptane, 2,3-dimethylbutane or cyclopentane; a nitroalkane such as nitromethane, nitroethane or nitropropane; a triazole such as 1,2,3-benzotriazole; an amine such as diethylamine, triethylamine, isopropylamine, butylamine or isobutylamine; an amylene such as α-, β- or γ-amylene or α- or β-isoamylene; an alcohol such as methanol, ethanol, n-propylalcohol, i-propylalcohol, n-butylalcohol, i-butylalcohol, s-butylalcohol or t-butylalcohol; a phosphite such as triphenyl phosphite or tributyl phosphite; an ether such as methyl cellosolve, tetrahydrofuran or 1,4-dioxane; an epoxide such as 1,2-butylene oxide or epichlorohydrin; a ketone such as acetone, methyl ethyl ketone or methyl butyl ketone; an ester such as ethyl acetate, propyl acetate or butyl acetate; a furan such as N-methylpyrrole; a chlorinated or brominated hydrocarbon such as dichloromethane, trichloroethane, trichloroethylene, tetrachloroethylene, trans-1,2-dichloroethylene, cis-1,2-dichloroethylene, 1-chloropropane, 2-chloro-2-methylpropane or 2-bromopropane; or a CFC hydrochlorofluorocarbon or hydrofluorocarbon other than those of the present invention, such as 1,1,2-trichlorotrifluoroethane, 1,1,2-trichloro-2,2-difluoroethane, tetrachloro-1,2-difluoroethane, 1,1-dichloro-2,2,2-trifluoroethane, 1,2-dichloro-1,1-difluoroethane, 1,2-dichloro-1-fluoroethane, 1,1-dichloro-1-fluoroethane, 1,1-dichloro-2,3,3,3-tetrafluoropropene-1, trans-3-chloro-1,1,1,2,4,4,5,5,5-nonfluoropentene-2, cis-3-chloro-1,1,1,2,4,4,5,5,5-nonafluoropentene-2 or 1,1,1,2,2,5,5,6,6,6-decafluorohexane.

The content of such additional components in the composition of the present invention is not particularly limited, but for the purpose of improving or controlling the solubility or obtaining a suitable boiling point or non-flammability, the content is usually from 0 to 50% by weight, preferably from 1 to 40% by weight. Nevertheless such incorporation will bring about an azeotropic or azeotropic-like composition. Further, to give the mixture a high level of stabilizing effect, it is effective to incorporate a stabilizer. The content of such additional components is usually from 1 ppm to 10% by weight, preferably from 10 ppm to 5% by weight. Further, the composition of the present invention may further contain various cleaning assistants, surfactants, emulsifying agents and water.

The hydrochlorofluoropropane azeotropic or azeotropic-like composition of the present invention is useful for various purposes, for example, as a blowing agent and so on, like conventional CFCs. It is particularly useful as a solvent, since it provides a solvency equivalent or superior to conventional R113. Specific applications as the solvent include a removing agent for flux, grease, oil, wax or ink, a coating solvent, an extracting agent, a cleaning or water-removing agent for various articles made of glass, ceramics, plastic, rubber or metal, particularly for semiconductor devices, electronic components, electronic circuit boards, electrical devices, precision machine parts or optical lenses. Further, it is useful as a resist developer, a resist-removing agent or a buff polishing and cleaning agent. As a cleaning method, manual wiping, dipping, spraying, shaking, ultrasonic cleaning or vapor cleaning may be employed.

Now, the present invention will be described in further detail with reference to Examples. However, it should be understood that the present invention is by no means restricted by such specific Examples.

### EXAMPLES 1

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225ca (boiling point: 51.1°C) | 60 |
| R225cb (boiling point: 56.1°C) | 10 |
| R244ca (boiling point: 54°C) | 30 |

As a result, 440 g of a distillation fraction was obtained. The fraction was analyzed by gas chromatography and was found to have the following composition.

| Composition | % by weight |
|---|---|
| R225ca | 58 |
| R225cb | 10 |
| R244ca | 32 |

### EXAMPLE 2

Using the composition of the present invention (R225ca/R225cb/R244ca = 58 wt%/10 wt%/32 wt%), a test for cleaning machine oil was conducted.

A SUS-304 test piece (25 mm x 30 mm x 2 mm in thickness) was immersed in machine oil (CQ-30, manufactured by Nippon Sekiyu K.K.) and then immersed for 5 minutes in the above composition of the present invention. As a result, it was confirmed that the machine oil can be removed satisfactorily as in the case of R113.

### EXAMPLE 3

Flammability of the composition of Example 2 (R225ca/R225cb/R244ca = 58 wt%/10 wt%/32 wt%) was measured with Tag closed cup flash point tester (JIS K2265), and the composition had no flash point and was confirmed to be non-flammable.

### EXAMPLE 4

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225ca (boiling point: 51.1°C) | 50 |
| R225cb (boiling point: 56.1°C) | 50 |

As a result, 320 g of a fraction at 54°C was obtained. The fraction was analyzed by gas chromatography and was found to have the following composition.

| Composition | % by weight |
|---|---|
| R225ca | 48 |
| R225cb | 52 |

### EXAMPLE 5

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225ca (boiling point: 51.1°C) | 80 |
| R225cb (boiling point: 56.1°C) | 20 |

As a result, 300 g of a fraction at 53°C was obtained. The fraction was analyzed by gas chromatography and was found to have the following composition.

| Composition | % by weight |
|---|---|
| R225ca | 79 |
| R225cb | 21 |

### EXAMPLE 6

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225ca (boiling point: 51.1°C) | 20 |
| R225cb (boiling point: 56.1°C) | 80 |

As a result, 290 g of a fraction at 55°C was obtained. The fraction was analyzed by gas chromatography and was found to have the following composition.

| Composition | % by weight |
|---|---|
| R225ca | 19 |
| R225cb | 81 |

### EXAMPLE 7

Using the composition of the present invention (R225ca/R225cb = 50 wt%/50 wt%), a test for cleaning machine oil was conducted.

A SUS-304 test piece (25 mm x 30 mm x 2 mm in thickness) was immersed in machine oil (CQ-30, manufactured by Nippon Sekiyu K.K.) and then immersed for 5 minutes in the above composition of the present invention. As a result, it was confirmed that the machine oil can be removed satisfactorily as in the case of R113.

### EXAMPLE 8

Flammability of the composition of the present invention (R225ca/R225cb = 50 wt%/50 wt%) was measured with Tag closed cup flash point tester (JIS K2265), and the composition had no flash point and was confirmed to be non-flammable.

### EXAMPLE 9

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225cb (boiling point: 56.1°C) | 50 |
| R244ca (boiling point: 54°C) | 50 |

As a result, 400 g of a fraction at 53°C was obtained. The fraction was analyzed by gas chromatography and was found to have the following composition.

| Composition | % by weight |
|---|---|
| R225cb | 47 |
| R244ca | 53 |

### EXAMPLE 10

Using the composition of the present invention (R225cb/R244ca = 47 wt%/53 wt%), a test for cleaning machine oil was conducted.

A SUS-304 test piece (25 mm x 30 mm x 2 mm in thickness) was immersed in machine oil (CQ-30, manufactured by Nippon Sekiyu K.K.) and then immersed for 5 minutes in the above composition of the present invention. As a result, it was confirmed that the machine oil can be removed satisfactorily as in the case of R113.

### EXAMPLE 11

Flammability of the composition of Example 10 (R225cb/R244ca = 47 wt%/53 wt%) was measured with Tag closed cup flash point tester (JIS K2265), and the composition had no flash point and was confirmed to be non-flammable.

### EXAMPLE 12

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225ca (boiling point: 51.1°C) | 70 |
| R244ca (boiling point: 54°C) | 30 |

As a result, 390 g of a fraction at 51°C was obtained. The fraction was analyzed by gas chromatography and was found to have the following composition.

| Composition | % by weight |
|---|---|
| R225ca | 72 |
| R244ca | 28 |

### EXAMPLE 13

Using the composition of the present invention (R225ca/R244ca = 72 wt%/28 wt%), a test for cleaning machine oil was conducted.

A SUS-304 test piece (25 mm x 30 mm x 2 mm in thickness) was immersed in machine oil (CQ-30, manufactured by Nippon Sekiyu K.K.) and then immersed for 5 minutes in the above composition of the present invention. As a result, it was confirmed that the machine oil can be removed satisfactorily as in the case of R113.

### EXAMPLE 14

Flammability of the composition of Example 13 (R225ca/R244ca = 72 wt%/28 wt%) was measured with Tag closed cup flash point tester (JIS K2265), and the composition had no flash point and was confirmed to be non-flammable.

### EXAMPLE 15

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225ca (boiling point: 51.1°C) | 80 |
| R243cc (boiling point: 60.2°C) | 20 |

As a result, 400 g of a fraction at 51°C was obtained. The fraction was analyzed by gas chromatography and was found to have the following composition.

| Composition | % by weight |
|---|---|
| R225ca | 76 |
| R243cc | 24 |

### EXAMPLE 16

Using the composition of Example 15 (R225ca/R243cc = 76 wt%/24 wt%), a test for cleaning machine oil was conducted.

A SUS-304 test piece (25 mm x 30 mm x 2 mm in thickness) was immersed in machine oil (CQ-30, manufactured by Nippon Sekiyu K.K.) and then immersed for 5 minutes in the above composition of the present invention. As a result, it was confirmed that the machine oil can be removed satisfactorily as in the case of R113.

### EXAMPLE 17

Flammability of the composition of Example 15 (R225ca/R243cc = 76 wt%/24 wt%) was measured with Tag closed cup flash point tester (JIS K2265), and the composition had no flash point and was confirmed to be non-flammable.

### EXAMPLE 18

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225cb (boiling point: 56.1°C) | 70 |
| R244cb (boiling point: 58°C) | 30 |

As a result, 450 g of a fraction at 56°C was obtained. The fraction was analyzed by gas chromatography and was found to have the following composition.

| Composition | % by weight |
|---|---|
| R225cb | 67 |
| R244cb | 33 |

### EXAMPLE 19

Using the composition of Example 18 (R225cb/R244cb = 67 wt%/33 wt%), a test for cleaning machine oil was conducted.

A SUS-304 test piece (25 mm x 30 mm x 2 mm in thickness) was immersed in machine oil (CQ-30, manufactured by Nippon Sekiyu K.K.) and then immersed for 5 minutes in the above composition of the present invention. As a result, it was confirmed that the machine oil can be removed satisfactorily as in the case of R113.

### EXAMPLE 20

1,000 g of a solvent composition having the following composition was charged to a distillation flask, and using a packed distillation column which contained approximately 20 theoretical plates, distillation was conducted under atmospheric pressure.

| Composition | % by weight |
|---|---|
| R225cb (boiling point: 56.1°C) | 60 |
| R243cc (boiling point: 60.2°C) | 40 |

As a result, 390 g of a fraction at 54°C was obtained. The fraction was analyzed by gas chromatography and was found to have the following composition.

| Composition | % by weight |
|---|---|
| R225cb | 63 |
| R243cc | 37 |

### EXAMPLE 21

Using the composition of Example 20 (R225cb/R244cc = 63 wt%/37 wt%), a test for cleaning machine oil was conducted.

A SUS-304 test piece (25 mm x 30 mm x 2 mm in thickness) was immersed in machine oil (CQ-30, manufactured by Nippon Sekiyu K.K.) and then immersed for 5 minutes in the above composition of the present invention. As a result, it was confirmed that the machine oil can be removed satisfactorily as in the case of R113.

The hydrochlorofluoropropane composition of the present invention has properties as excellent as the conventional CFCs and has an azeotropic composition of an excellent azeotropic-like properties. Therefore, it shows no substantial change during boiling or evaporating. It can be used in the same manner as a conventional single CFC and thus has a merit that no substantial change of the conventional technique is required for its use.

## Claims

1. An azeotropic or azeotropic-like composition which comprises 3,3-dichloro-1,1,1,2,2-pentafluoropropane (R225ca), 1,3-dichloro-1,1,2,2,3-pentafluoropropane (R225cb) and 3-chloro-1,1,2,2-tetrafluoropropane (R244ca).

2. The composition according to Claim 1, which comprises from 12 to 92% by weight of R225ca, from 3 to 77% by weight of R225cb and from 3 to 69% by weight of R244ca.

3. The azeotropic composition according to claim 1, which comprises 58% by weight of R225ca, 10% by weight of R225cb and 32% by weight of R244ca.

4. An azeotropic or azeotropic-like composition which comprises 3,3-dichloro-1,1,1,2,2-pentafluoropropane (R225ca) and 1,3-dichloro-1,1,2,2,3-pentafluoropropane (R225cb) in weight ratios of 80/20 to 19/81.

5. An azeotropic or azeotropic-like composition which comprises 1,3-dichloro-1,1,2,2,3-pentafluoropropane (R225cb) and 3-chloro-1,1,2,2-tetrafluoropropane (R244ca).

6. The composition according to Claim 5 , which comprises from 27 to 67% by weight of R225cb and from 33 to 73% by weight of R244ca.

7. The composition according to Claim 5, which comprises 47% by weight of R225cb and 53% by weight of R244ca.

8. An azeotropic or azeotropic-like composition which comprises 3,3-dichloro-1,1,1,2,2-pentafluoropropane (R225ca) and 3-chloro-1,1,2,2-tetrafluoropropane (R244ca).

9. The composition according to Claim 8 , which comprises from 52 to 92% by weight of R225ca and from 8 to 48% by weight of R244ca.

10. The composition according to Claim 8, which comprises 72% by weight of R225ca and 28% by weight of R244ca.

11. An azeotropic or azeotropic-like composition, which comprises from 56 to 96% by weight of R225ca and from 4 to 44% by weight of R243cc.

12. The azeotropic composition according to claim 11, which comprises 76% by weight of R225ca and 24% by weight of R243cc.

13. An azeotropic or azeotropic-like composition, which comprises from 47 to 87% by weight of R225cb and from 13 to 53% by weight of R244cb.

14. The azeotropic composition according to claim 13, which comprises 67% by weight of R225cb and 33% by weight of R244cb.

15. An azeotropic or azeotropic-like composition, which comprises from 43 to 83% by weight of R225cb and from 17 to 57% by weight of R243cc.

16. The azeotropic composition according to claim 15, which comprises 63% by weight of R225cb and 37% by weight of R243cc.

## Patentansprüche

1. Azeotrope oder azeotropähnliche Zusammensetzung, umfassend 3,3-Dichlor-1,1,1,2,2-pentafluorpropan (R225ca), 1,3-Dichlor-1,1,2,2,3-pentafluorpropan (R225cb) und 3-Chlor-1,1,2,2-tetrafluorpropan (R244ca).

2. Zusammensetzung nach Anspruch 1, umfassend 12 bis 92 Gew.-% R225ca, 3 bis 77 Gew.-% R225cb und 3 bis 69 Gew.-% R244ca.

3. Azeotrope Zusammensetzung nach Anspruch 1, umfassend 58 Gew.-% R225ca. 10 Gew.-% R225cb und 32 Gew.-% R244ca.

4. Azeotrope oder azeotropähnliche Zusammensetzung, umfassend 3,3-Dichlor-1,1,1,2,2-pentafluorpropan (R225ca) und 1,3-Dichlor-1,1,2,2,3-pentafluorpropan (R225cb) in Gewichtsverhältnissen von 80/20 bis 19/81.

5. Azeotrope oder azeotropähnliche Zusammensetzung, umfassend 1,3-Dichlor-1,1,2,2,3-pentafluorpropan (R225cb) und 3-Chlor-1,1,2,2-tetrafluorpropan (R244ca).

6. Zusammensetzung nach Anspruch 5, umfassend 27 bis 67 Gew.-% R225cb und 33 bis 73 Gew.-% R244ca.

7. Zusammensetzung nach Anspruch 5, umfassend 47 Gew.-% R225cb und 53 Gew.-% R244ca.

8. Azeotrope oder azeotropähnliche Zusammensetzung, umfassend 3,3-Dichlor-1,1,1,2,2-pentafluorpropan (R225ca) und 3-Chlor-1,1,2,2-tetrafluorpropan (R244ca).

9. Zusammensetzung nach Anspruch 8, umfassend 52 bis 92 Gew.-% von R225ca und 8 bis 48 Gew.-% R244ca.

10. Zusammensetzung nach Anspruch 8, umfassend 72 Gew.-% R225ca und 28 Gew.-% R244ca.

11. Azeotrope oder azeotropähnliche Zusammensetzung, umfassend 56 bis 96 Gew.-% R225ca und 4 bis 44 Gew.-% R243cc.

12. Azeotrope Zusammensetzung nach Anspruch 11, umfassend 76 Gew.-% R225ca und 24 Gew.-% R243cc.

13. Azeotrope oder azeotropähnliche Zusammensetzung, umfassend 47 bis 87 Gew.-% R225cb und 13 bis 53 Gew.-% R244cb.

14. Azeotrope Zusammensetzung nach Anspruch 13, umfassend 67 Gew.-% R225cb und 33 Gew.-% R244cb.

15. Azeotrope oder azeotropähnliche Zusammensetzung, umfassend 43 bis 83 Gew.-% R225cb und 17 bis 57 Gew.-% R243cc.

16. Azeotrope Zusammensetzung nach Anspruch 15, umfassend 63 Gew.-% R225cb und 37 Gew.-% R243cc.

## Revendications

1. Composition azéotropique ou de type azéotropique, qui comprend du 3,3-dichloro-1,1,1,2,2-pentafluoropropane (R225ca), du 1,3-dichloro-1,1,2,2,3-pentafluoropropane (R225cb) et du 3-chloro-1,1,2,2-tétrafluoropropane (R244ca).

2. Composition selon la revendication 1, qui comprend de 12 à 92 % en poids de R225ca, de 3 à 77 % en poids de R225cb et de 3 à 69 % en poids de R244ca.

3. Composition azéotropique selon la revendication 1, qui comprend 58 % en poids de R225ca, 10 % en poids de R225cb et 32 % en poids de R244ca.

4. Composition azéotropique ou de type azéotropique qui comprend du 3,3-dichloro-1,1,1,2,2-pentafluoropropane (R225ca) et du 1,3-dichloro-1,1,2,2,3-pentafluoropropane (R225cb) selon un rapport pondéral de 80/20 à 19/81.

5. Composition azéotropique ou de type azéotropique qui comprend du 1,3-dichloro-1,1,2,2,3-pentafluoropropane (R225cb) et du 3-chloro-1,1,2,2-tétrafluoropropane (R244ca).

6. Composition selon la revendication 5, qui comprend de 27 à 67 % en poids de R225cb et de 33 à 73 % en poids de R244ca.

7. Composition selon la revendication 5, qui comprend 47 % en poids de R225cb et 53 % en poids R244ca.

8. Composition azéotropique ou de type azéotropique, qui comprend du 3,3-dichloro-1,1,1,2,2-pentafluoropropane (R225ca) et du 3-chloro-1,1,2,2-tétrafluoropropane (R244ca).

9. Composition selon la revendication 8, qui comprend de 52 à 92 % en poids de R225ca et de 8 à 48 % en poids de R244ca.

10. Composition selon la revendication 8, qui comprend 72 % en poids de R225ca et 28 % en poids de R244ca.

11. Composition azéotropique ou de type azéotropique, qui comprend de 56 à 96 % en poids de R225ca et de 4 à 44 % en poids de R243cc.

12. Composition azéotropique selon la revendication 11, qui comprend 76 % en poids de R225ca et 24 % en poids de R243cc.

13. Composition azéotropique ou de type azéotropique, qui comprend de 47 à 87 % en poids de R225cb et de 13 à 53 % en poids de R244cb.

14. Composition azéotropique selon la revendication 13, qui comprend 67 % en poids de R225cb et 33 % en poids de R244cb.

15. Composition azéotropique ou de type azéotropique, qui comprend de 43 à 83 % en poids de R225cb et de 17 à 57 % en poids de R243cc.

16. Composition azéotropique selon la revendication 15, qui comprend 63 % en poids de R225cb et 37 % en poids de R243cc.
